# EUROPEAN PATENT SPECIFICATION

(11) **EP 2 739 215 B1**
(45) Date of publication and mention of the grant of the patent: **01.05.2019**
(21) Application number: 12746239.8
(22) Date of filing: 03.08.2012
(51) Int. Cl.: A61B 17/12, A61L 31/00, A61L 31/14, A61B 17/00, A61B 50/30, A61B 90/00

(54) **POLYMER-BASED OCCLUSION DEVICES AND SYSTEMS**
POLYMER BASIERTE OKKLUSIONSVORICHTUNGEN UND SYSTEME
DISPOSITIFS ET SYSTÈMES D'OCCLUSION À BASE DE POLYMÈRE

(30) Priority: 05.08.2011 US 201161515753 P; 02.08.2012 US 201213565707
(43) Date of publication of application: 11.06.2014
(73) Proprietor: W.L. Gore & Associates, Inc., Newark, DE 19711 (US)
(72) Inventor: CHU, Chaokang, Hockessin, Delaware 19701 (US); CLEEK, Robert, L., Flagstaff, Arizona 86004 (US); CULLY, Edward, H., Flagstaff, Arizona 86004 (US); DUNCAN, Jeffrey, Flagstaff, Arizona 86001 (US); PIETRZAK, Krzysztof, R., Flagstaff, Arizona 86001 (US); SHAW, Edward Emil, Flagstaff, Arizona 86004 (US); VONESH, Michael, J., Flagstaff, Arizona 86004 (US); ZACHARIAS, Eric, H., Flagstaff, Arizona 86004 (US)
(74) Representative: HGF Limited
(86) International application number: PCT/US2012/049465
(87) International publication number: WO 2013/022736

(56) References cited:
- WO-A1-2008/147861
- WO-A1-2009/009466
- WO-A1-2009/070686
- WO-A1-2009/132141
- WO-A2-97/19643
- WO-A2-2006/047748
- WO-A2-2007/073549
- US-A1- 2002 026 217
- US-A1- 2002 052 653
- US-A1- 2003 181 931
- US-A1- 2005 085 836
- US-A1- 2005 090 861
- US-A1- 2005 133 046
- US-A1- 2006 178 696
- US-B1- 6 440 098
- US-B1- 6 589 199

## Description

### Cross-Reference to Related Applications

This application claims priority to U.S. Application No. 13/565,707, filed August 2, 2012, which claims the benefit of Provisional Application No. 61/515,753, filed August 5, 2011.

### Technical Field

The disclosure relates to devices, systems and methods for occlusion.

### Background

The need for filling, embolizing, or occluding (which terms may be used herein interchangeably) arises in various settings. As it relates to the cardiovascular system, distension of the vessel wall creates aneurysms that can range in size from small (e.g., intracranial "berries") to large (e.g., distension of the aortic wall). Aneurysms relatively frequently involve side branch vessels and can be convoluted. Irrespective of the size and location, aneurysms can be problematic and often require occlusion.

Other clinical applications involving therapeutic occlusion or embolization include treatment of arterio-venous malformations, hemorrhagic stroke, vascular trauma and/or perforation, and closure of mural defects in the cardiovascular system.

Another application wherein occlusion is useful is in the treatment of cancer. For example, interventional oncology often necessitates the occlusion of vasculature to "starve" diseased tissue (e.g., a tumor) and additionally, to isolate therapeutic agents in contact with diseased tissue.

The need for occlusion may also arise in the context of endovascular devices, for example stents, stent grafts, heart valves, implants, catheters, etc. Often an incomplete seal exists between an endovascular device and surrounding tissue, for example, in the case of an incomplete endovascular deployment or as a result of the irregular tissue deformation created by an aneurysm. An incomplete seal may also be found between a plurality of endovascular devices. Such "endoleaks" often require occlusion.

Various approaches to occlusion exist in the prior art. For example, embolic coils of metallic wire (e.g., platinum) and PET (e.g., Dacron) are deployed from stent-crossing catheters to occlude aneurysms. Mechanical vessel occluders and liquid embolic agents are also known and used, for example, to sequester diseased tissue.

Yet, the existing approaches may suffer from drawbacks. For example, metallic coils may not be suitable for smaller applications and are not radiographically transparent, maximum sequestration of diseased tissue is often difficult to achieve, and no proven technique exists for occlusion of certain types of endoleaks.

What is therefore needed in the art is a radiographically transparent or temporarily radio-opaque occluder suitable for use in connection with aneurysms of all sizes, sequestration of diseased tissue, and endoleaks, to name just a few applications. What is also needed in the art is an occluder having porous properties. The present disclosure addresses these needs and others. A system for occluding according to the preamble of claim 1 is disclosed in US 6,440,098 B1.

### SUMMARY

This specification describes devices, systems, and processes for endovascular occlusion, such as occlusion of blood vessel aneurysms. In brief, various embodiments are disclosed whereby a catheter delivers a first material to occlude an endovascular space. Optionally, the catheter may also be used to deliver a second material to combine with and expand the first material, while the first material is disposed in the endovascular space to be occluded. A system for occluding according to the invention is defined in claim 1. Preferred embodiments of the invention are defined in the dependent claims. The aspects recited in the following paragraphs do not necessarily form part of the invention.

In a first general aspect, an occlusion device includes an elongate element having a first configuration and a second configuration, where the first configuration has a relatively low crossing profile and the second configuration is tumbled. The elongate element includes at least one modification to increase the surface area, surface drag, and/or axial profile of the elongate element, and the elongate element has a first volume in the first configuration and a second volume greater than the first volume in the second configuration after an administration of an alginate.

In a second general aspect, a system for occluding includes an elongate element located within a lumen of a catheter, wherein the elongate element is configured to be delivered to a site for occlusion upon hydraulic flow through the lumen of the catheter. The elongate element is porous and imbibed with at least one of a therapeutic composition, swellable agent, bioactive agent, drug, or compound. The elongate element comprises a first configuration suitable for delivery, and a second configuration suitable for occlusion.

In a third general aspect, a method of occluding includes imbibing a porous elongate element comprised of ePTFE with a calcium-containing solution, delivering, via a delivery catheter, the calcium-imbibed porous elongate element to a target occlusion site, and administering, after the calcium-imbibed porous elongate element has been completely delivered to the target occlusion site and resides entirely within a volume defined by the target occlusion site, an alginate-containing solution to the target occlusion site.

In a fourth general aspect, a method of occluding includes accessing a target occlusion site with a catheter that includes a plurality of lumens, each of the lumens housing a separate elongate element. The method also includes delivering to the target occlusion site via a first lumen of the plurality of lumens, a first elongate element. The method further includes delivering to the target occlusion site via a second lumen of the plurality of lumens, a second elongate element.

In a fifth general aspect, a system for occluding includes a catheter that comprises a proximal end, a distal end, and a delivery lumen that extends from the proximal end to a location at or near the distal end. The system also includes a dispenser that comprises a plurality of dispensing lumens, each of the dispensing lumens housing a separate elongate element, wherein the dispenser is positionable with respect to the proximal end of the catheter such that, for each dispensing lumen of the plurality of dispensing lumens, the dispenser may be positioned so that the respective dispensing lumen is in fluid communication with the delivery lumen of the catheter.

In a sixth general aspect, a method of occluding includes delivering a first elongate element to a target occlusion site, the first elongate element delivered via a delivery lumen of a delivery catheter from a first dispensing lumen of a dispenser comprising a plurality of dispensing lumens. The method also includes positioning the dispenser to align a second dispensing lumen of the plurality of dispensing lumens with a proximal end of the delivery lumen. The method further includes delivering a second elongate element to the target occlusion site via the delivery lumen of the delivery catheter from the second dispensing lumen.

According to various aspects of the invention, an elongate element comprises at least a first configuration suitable for delivery and a second configuration suitable for occlusion. In example embodiments, the elongate element is biased toward the second configuration, while according to the invention, the elongate element has no bias such that the second configuration is random.

According to the invention, the elongate element is porous to imbibe one or more of a reagent, therapeutic composition, agent, drug, or compound.

Example embodiments of the present invention comprise an elongate element modified to promote a desired therapeutic response like thrombogenesis, assist in increased hemostatic properties, allowing volumetric changes to the elongate element, and/or enhance its delivery or deployment by, for example, adjusting a dimensional characteristic. The elongate element may be imbibed with a first reagent that serves to activate or react with a second reagent. The second reagent may be used to hydraulically deliver the elongate element. The elongate element of the present invention may be delivered over a catheter or out from within a catheter. The elongate element of the present invention may also be surgically implanted and activated in situ. Further, the elongate element may be incorporated into an implantable prosthesis.

Other aspects, features, and advantages will be apparent from the description, the drawings, and the claims.

### BRIEF DESCRIPTION OF THE DRAWINGS

Figure 1 illustrates an example elongate element in a delivery configuration;
Figure 2 illustrates an example elongate element in a occlusion configuration;
Figure 3 illustrates an example elongate element having fibers adhered to its surface;
Figure 4A-4B illustrate an example elongate element having an auger configuration;
Figure 5A-5B illustrate an example elongate element having a spiral configuration;
Figure 6 illustrates an example elongate element comprising a plurality of beads;
Figure 7A illustrates an example delivery system comprising a stent-crossing catheter;
Figure 7B illustrates an example delivery system comprising a catheter supported by a stent device;
Figure 7C illustrates an example delivery system comprising a catheter supported by a balloon device;
Figure 8A-8B illustrate an example delivery device wherein the elongate element is tubular;
Figure 9A-9D illustrate various embodiments for sealing an example elongate element having a tubular configuration;
Figure 10 illustrates an example distensible elongate element;
Figure 11A illustrates another example embodiment of a delivery system;
Figure 11B illustrates another example embodiment of a delivery system;
Figure 12A-12C illustrate another example embodiment of a delivery system;
Figures 13A-13B illustrate an example embodiment of an occlusion device comprising multiple types of elongate elements;
Figure 14 illustrates another example embodiment of a delivery system;
Figure 15 illustrates an example embodiment of an elongate element cutting device;
Figure 16 illustrates another example embodiment of an elongate element cutting device;
Figure 17 illustrates another example embodiment of an elongate element cutting device;
Figure 18 illustrates another example embodiment of an elongate element cutting device;
Figures 19A-19D illustrate additional example embodiments of an elongate element cutting device; and
Figure 20 illustrates an example embodiment of a method for performing endovascular occlusion.

Like reference symbols in the various drawings indicate like elements.

### DETAILED DESCRIPTION OF THE ILLUSTRATED EMBODIMENTS

Persons skilled in the art will readily appreciate that various aspects of the present disclosure may be realized by any number of methods and apparatuses configured to perform the intended functions. Stated differently, other methods and apparatuses may be incorporated herein to perform the intended functions. It should also be noted that the accompanying drawing figures referred to herein are not all drawn to scale, but may be exaggerated to illustrate various aspects of the depicted embodiments, and in that regard, the drawing figures should not be construed as limiting. Finally, although the embodiments may be described in connection with various principles and beliefs, the embodiments should not be bound by theory. In the following, the unit inch is used with the abbreviations in and ". 1 inch amounts to 2,54 cm.

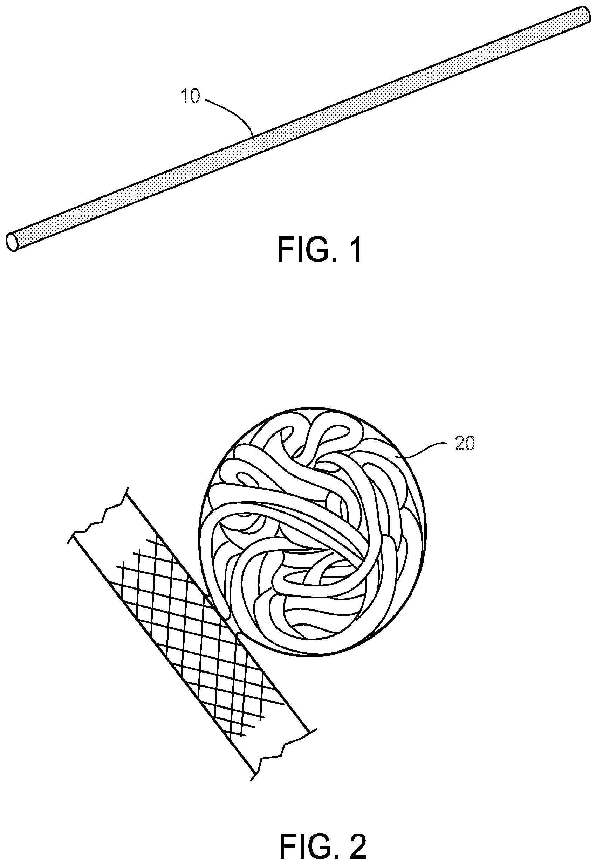

With reference now to FIG. 1, an occluder in accordance with some embodiments provided herein comprises an elongate element 10, which is a biocompatible material. In various embodiments, elongate element 10 may have a solid cross section (e.g., a single filament), have multiple filaments, or have a tubular structure (e.g., a micro-tube). Other embodiments of the elongate element may also include a fabric or a membrane. Elongate element 10 may be comprised of a single material or a plurality of materials, for example, having a solid or tubular core and one or more surrounding layers. In the case of an elongate element having a tubular structure, the elongate element may be sealed at one end or at both ends, for example, as described herein. Similarly, for embodiments incorporating a tubular structure, one or both ends may incorporate a valve apparatus.

Those skilled in the art will appreciate that example elongate elements may vary dimensionally depending on the particular application. However, an example embodiment of an elongate element having a solid cross section may have a diameter of from about 0.005in to about 0.500in, preferably about 0.020in (about 0.5mm). Similarly, an example embodiment of an elongate element having a tubular structure may have an outer diameter of from about 0.005in to about 0.500in, preferably about 0.020in (about 0.5mm), and an inner diameter of from about 0.001in to about 0.100in, preferably about 0.015in. In terms of length, example elongate elements may be from about 1.0in to about 20.0in, preferably about 6.0in. In terms of volume occupying capacity, example elongate elements in their second configuration may occupy from less than or about 0.5mL (e.g., in the case of cerebral aneurysms) to more than or about 250mL (e.g., in the case of aortic aneurysms). Again, however, elongate elements may be smaller or larger depending on the particular application.

Moreover, while the foregoing embodiments have been described in terms of diameter, those skilled in the art will appreciate that example elongate elements may vary cross-sectionally depending on the particular application. More specifically, elongate elements can have any cross-sectional shape including but not limited to profiles that are circular, oval, triangular, square, polygon shaped or randomly shaped. Additionally, it is understood that cross-sectional shape may vary as a function of activation or deployment.

In some embodiments, a cross-sectional dimension of the elongate element can be varied along its length. For example, an elongate element with a circular cross-section can have a different diameter at different positions along the length of the elongate element (e.g., resulting in a tapered profile). Varying the cross-sectional dimension in a portion of the elongate element can generally affect the flexibility and column strength of the elongate element in that portion. For instance, a portion of a circular cross-sectional elongate element with a reduced diameter can generally be more flexible and have a lower column strength than a portion that has a larger diameter (assuming all other factors are constant).

Example embodiments comprise an elongate element that exhibits flexibility. Example embodiments comprise an elongate element having a low column strength, for example, so as to "tumble" on itself (as that term is defined herein), and thereby not pierce a vessel wall, when coming into contact with a vessel wall. In some embodiments, elongate element 10 can exhibit greater flexibility and lower column strength at its trailing-end portion than at its leading-end portion. The purpose of more flexibility at the trailing-end can be to enable the last portion entering a space to more easily "tumble" on itself, thereby helping the elongate element to more thoroughly fill the space as the open space become smaller. Greater flexibility at the trailing-end portion can be achieved, for example, by reducing the cross-sectional size of the trailing-end portion, or by varying the modulus of elasticity of the elongate element along its length while maintaining a consistent cross-sectional size (or by a combination of such factors). Still other example embodiments comprise a distensible elongate element, or in other words, an elongate element that is capable of radial stretching or expansion. The elongate element is porous in various example embodiments, and may be non-metallic and/or radiographically transparent.

Some embodiments of elongate element 10 can include particular weakened regions to facilitate the shearing, tearing, fracturing, severing, or otherwise separating of elongate element 10. This feature can be useful, for example, when the space being occluded has received the proper amount of elongate element 10, and elongate element 10 can then be severed to stop further delivery.

Suitable materials for use in connection with an example elongate element may comprise polymers, such as a fluoropolymer like expanded polytetrafluoroethylene ("ePTFE"). For example, U.S. Patent 5,814,405 to Branca et al. describes a suitable ePTFE material. However, those skilled in the art will appreciate that any materials may be used that exhibit the desired properties described herein, for example, nylons, polycarbonates, polyethylenes, polypropylenes, as well as combinations or sub-combinations thereof.

In embodiments comprising ePTFE, the fibril and node openness (i.e., the ePTFE's permeability or porosity) may be selected for optimum wettability. In example embodiments, the elongate element comprises a plurality of concentric layers differing in their wettability. In example embodiments, the elongate element's permeability is selected to enable flushing of air, prior to insertion. In this regard, the elongate element may be permeable to saline but not to a reagent or therapeutic agent (e.g., one or more reagent or therapeutic agent imbibed or enclosed within the elongate element) so as to not unintentionally flush said reagent or therapeutic agent. The elongate element in example embodiments comprises at least a first configuration suitable for delivery and a second configuration suitable for occlusion. For example, with reference again to FIG. 1, the first configuration may be selected to minimize the crossing profile of elongate element 10. In that regard, the first configuration may be linear or curved, for example to facilitate delivery of elongate element 10 together with an endovascular device through tortuous passageways.

In contrast, and with reference to FIG. 2, the second configuration of an elongate element 20 may be selected to maximize filling or occlusion and may be characterized as "tumbled," which as used herein means tumbled, folded, coiled, wrinkled, twisted, crumpled, combinations of the foregoing, and the like. In example embodiments, elongate element 20 is biased toward the second configuration, for example materially or structurally biased, or biased as the result of any of the modifications to elongate element 20 described herein. In other embodiments however, elongate element 20 has no bias such that the second configuration is random, as illustrated in FIG. 2.

In some embodiments, the elongate element may be removable following implantation. In this regard, the elongate element may comprise a third configuration suitable for removal. Not unlike the first configuration, the third configuration may be selected to minimize the crossing profile of the elongate element, for example to facilitate removal of the elongate element together with an endovascular device through tortuous passageways.

In some embodiments, the elongate element may be configured to return to a first configuration suitable for removal from a second configuration suitable for occlusion. In this regard, the first and third configurations may be substantially the same.

In example embodiments, the elongate element is porous to imbibe a reagent or any other material. As used herein, "imbibe" means to absorb, take in, or otherwise assimilate. The reagents or materials can include therapeutic compositions, bioactive agents, drugs, or compounds, including but not limited to: small molecule drugs; large molecule drugs; medicaments; cardiovascular agents; sclerotic agents; chemotherapeutics; antimicrobials; antibiotics (e.g., dactinomycin (actinomycin O) daunorubicin, doxorubicin, and idarubicin), anthracyclines, mitoxantrone, bleomycins, plicamycin (mithramycin) and mitomycin); anesthetics; alkaloids (nicotine); hemostatics; antihistamines; antitumor agents; antilipids; antifungals; antimycotics; antipyretics; antirestenotics (e.g., pimecrolimus, cytochalasin, dicumarol, cyclosporine, latrunculin A, methotrexate, tacrolimus, halofuginone, mycophenolic acid, genistein, batimistat, dexamethasone, cudraflavone, simvastatin, prednisolone, doxorubicin, bromopyruvic acid, cilostazol, carvedilol, mitoxantrone, tranilast, etoposide, hirudin, trapidil, mitomycin C, abciximab, cilostazol, irinotecan, estradiol, diaziquone, dipyridamole, melatonin, colchicine, nifedipine, vitamin E, paclitaxol, diltiazem, vinblastine, verapamil, vincristine, rapamycin (e.g., Albumin-Bound (Nab)-Rapamycin (Abraxane), angiopeptin, everolimus, heat shock proteins, zotarolimus, nitroglycerin, prednisone); antimitotics/antiproliferatives (e.g., including natural products such as vinca alkaloids (e.g., vinblastine, vincristine, and vinorelbine), paclitaxel, epidipodophyllotoxins (e.g., etoposide, teniposide), alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes - dacarbazinine (DTIC)); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (e.g., estrogen); vasodilators; hypertensive agents; oxygen free radical scavengers; vitamins; antivirals; analgesics; antiinflammatories (e.g., adrenocortical steroids (cortisol, cortisone, fludrocortisone, prednisone, prednisolone, 6a-methylprednisolone, triamcinolone, betamethasone, and dexamethasone, dexamethasone sodium phosphate, dexamethasone acetate, beclomethasone dipropionate); non-steroidal agents (e.g., salicylic acid derivatives such as aspirin); para-aminophenol derivatives e.g., acetominophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone; gold compounds (auranofin, aurothioglucose, gold sodium thiomalate); diagnostic agents; visualization agents; angiographic contrast agents; peptides; proteins; antibodies (e.g., britumomab (Zevalin), bevacizumab (Avastin), rituximab (Rituxan), Cetuximab (Erbitux), Ofatumumab (Arzerra), Panitumumab (Vectibix), Trastuzumab (Herceptin), and Tositumomab (Bexxar)); enzymes (e.g., L-asparaginase); antiplatelet agents (such as G(GP)llbllla inhibitors and vitronectin receptor antagonists); insulin; phase contrast agents, and radio-opaque agents; thrombolytics intended to facilitate the breakup of thrombus; anticoagulants (e.g., heparin, synthetic heparin salts and other inhibitors of thrombin), intended to prevent thrombosis; fibrinolytic agents (such as tissue plasminogen activator, streptokinase and urokinase), aspirin, dipyridamole, ticlopidine, clopidogrel, abciximab; antimigratories; antisecretories (e.g., breveldin); immunosuppressives: (cyclosporine, tacrolimus (FK-S06), sirolimus (rapamycin), azathioprine, mycophenolate mofetil); angiogenic agents: vascular endothelial growth factor (VEGF), fibroblast growth factor (FGF); angiotensin receptor blocker; nitric oxide donors; anti-sense oligionucleotides and combinations thereof; cell cycle inhibitors, mTOR inhibitors, and growth factor signal transduction kinase inhibitors; RNA; viruses; and combinations thereof.

Example embodiments comprise an elongate element modified to promote a desired therapeutic response like thrombogenesis, assist in increased hemostatic properties and/or enhance its delivery or deployment by, for example, adjusting a dimensional characteristic. As used herein, "adjusting" means increasing, decreasing, maximizing, minimizing, or otherwise optimizing. As used herein, "dimensional characteristic" may comprise surface area, surface drag, volume and/or axial profile. Example modifications may be made to the elongate element in its first and/or its second configuration.

By way of non-limiting example, in a preferred embodiment, the volume of an elongate element in its first configuration is less than needed to completely occlude a target occlusion space, but its volume in its second configuration substantially completely occludes or otherwise occupies the space. For example, the tumbled elongate element may only fill 80% of the space to be occluded, but through gelation or cross-linking it may fill approaching 100% of the space to be occluded.

In this regard, a preferred embodiment comprises imbibing the elongate element with a first reagent that serves to activate or react (e.g., to form a gel or to cross-link) with a second reagent. In a preferred embodiment, the first and second reagents are a calcium chloride solution and a sodium alginate solution, respectively. For example, the elongate element 10 can be imbibed with a solution of approximately 10% calcium chloride. After delivery of the imbibed elongate element to the endovascular occlusion site, a solution of approximately 1.6-1.8 wt% sodium alginate can be delivered to the occlusion site to thereby form a gel with the elongate element in situ. In another example embodiment, the elongate element 10 can be imbibed with sodium alginate, and the calcium chloride solution can be subsequently provided to form a gel with the elongate element in situ. In another example embodiment, the elongate element 10 can be imbibed with a block copolymer that can be cross-linked with polyvalent compounds, and the second reagent can be a polyvalent compound.

Alginic acid (alginate) is an example block copolymer consisting of glucuronic acid and mannuronic acid that can be cross-linked with polyvalent compounds. Polyvalent compounds that may be used include divalent or trivalent metal salts such as barium, lead, copper, strontium, cadmium, calcium, zinc, nickel, cobalt, manganese, iron and magnesium. Polyvalent compounds may also include cationic polymers such as polyethyleneimine, poly-L-lysine, diethylaminoethyl dextran, polyvinylamine, chitosan, and poly(allylamine). A combination of polyvalent cations and/or cationic polymers may also be used to cross-link the block copolymer.

The second reagent may also be used to hydraulically deliver the elongate element, as described herein. The product of the first and second reagents may promote a desired therapeutic response like thrombogenesis, assist in increased hemostatic properties and/or enhance delivery of the elongate element by, for example, increasing the volume or otherwise adjusting a dimensional characteristic of the elongate element in its second configuration through gelation or cross-linking, to name a few examples. In some embodiments a non-reactive liquid (e.g., saline) can be used to hydraulically deliver the elongate element.

Various compounds can be incorporated into a gel according to example embodiments, including the materials described herein and compounds that may be especially relevant for hemostatic applications, for example, collagen, oxidized regenerated cellulose, gelatin, thrombin, fibrin and/or fibrin sealants, and/or synthetic sealants (e.g. crosslinked PEG, cyanoacrylate, etc.). Combinations of these compounds may also be used.

Fibrous composites may be formed by combining a gel according to example embodiments and/or crosslinked polymer compositions with fibrous materials. Said fibrous materials may be ceramic, inorganic, metallic or polymeric. Example ceramic and inorganic materials include, but are not limited to, alumina, alumina silicate, bismuth titanate, boron nitride, calcium phosphate, carbon, carbon nanotubes, glass, graphite, hydroxyapatite, lead metaniobate, lead nickel niobate, lead zirconate titanate, lithium aluminate, oxide nanotubes, silicon carbide, silicone nitride, tin oxide, titanium dioxide, yttrium aluminum garnet, zirconium diboride, and combinations thereof. Example metallic fibrous materials include, but are not limited to, aluminum, copper, gold, iron, magnesium, nickel-titanium, platinum, silver, steel, alloys thereof, and combinations thereof. Example polymeric fibrous materials include, but are not limited to, cellulose, cellulosic derivatives (e.g., carboxymethylcellulose and hydroxyethylcellulose), chitin, chitosan, collagen, fluoropolymers, polyacrylates, polyamides, polyanhydrides, polyesteramides, polyesters, polyesterurethanes, polyetheramides, polyetheresters, polyetheresterurethanes, polymethacrylates, polyolefins, polyurethanes, polyvinylalcohol, and combinations thereof.

In embodiments comprising a tubular elongate element, its lumen in a first configuration may be substantially patent (i.e., open) and its lumen in a second configuration may be substantially non-patent (i.e., obstructed). By way of non-limiting example, the lumen of a tubular elongate element may be obstructed with a gel, as described herein, in its second configuration.

Other modifications may be made to the elongate element, alone or in combination, to adjust a dimensional characteristic and thereby promote a desired therapeutic response like thrombogenesis, assist in increased hemostatic properties and/or enhance its delivery or deployment.

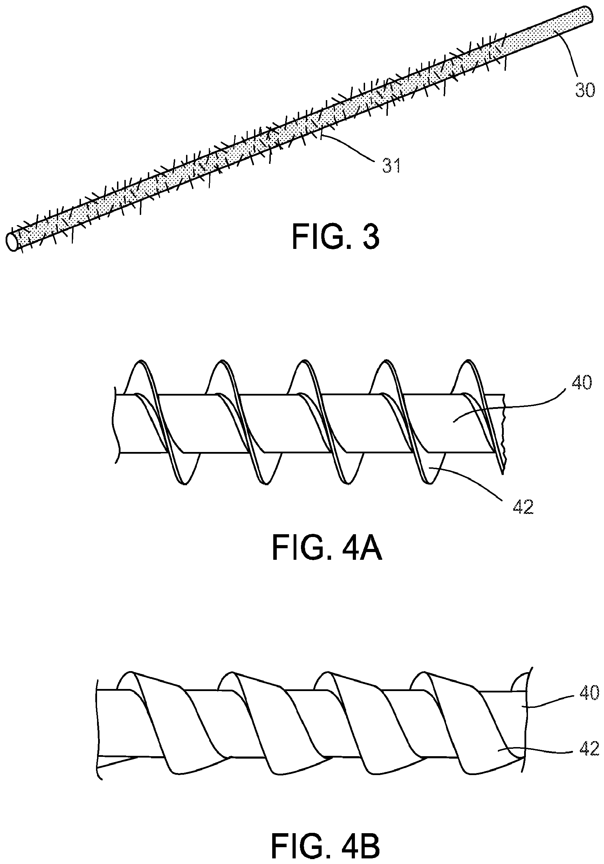

In this regard, mechanical roughening and/or plasma treating may contribute to an increased surface area or otherwise adjusted dimensional characteristic. Similarly, adherence of fibers 31 (e.g., ePTFE, Dacron, etc.) to the surface of an elongate element 30 may be used, for example, as shown in FIG. 3.

Bioabsorbable polymer coatings (e.g., bioabsorbable non-woven self-cohered web materials, such as disclosed in U.S. Patent No. 7,659,219), swelling hydrogel coatings, as well as heat treatments (e.g., methods to coalesce ePTFE fibrils and leave nodes standing) may also be used to provide to an adjusted dimensional characteristic. The wettability of the elongate element may also be altered in example embodiments (e.g., with PVA to make an ePTFE elongate element more hydrophilic). See U.S. Patent No. 5,874,165, for examples of imbibing ePTFE with a hydrogel.

With reference now to FIGS. 4A-4B, an auger configuration may be used to provide to an increased surface area or otherwise adjusted dimensional characteristic. By way of non-limiting example, and with reference to U.S. Publication No. 2009/0198219, a polymer film 42 may be helically wrapped upon an elongate element 40. By adhering polymer film 42 only along one edge, configurations as shown in FIGS. 4A-4B may be devised. Configurations as such may assist in minimizing friction within a delivery system while also providing adequate seal for hydraulic delivery.

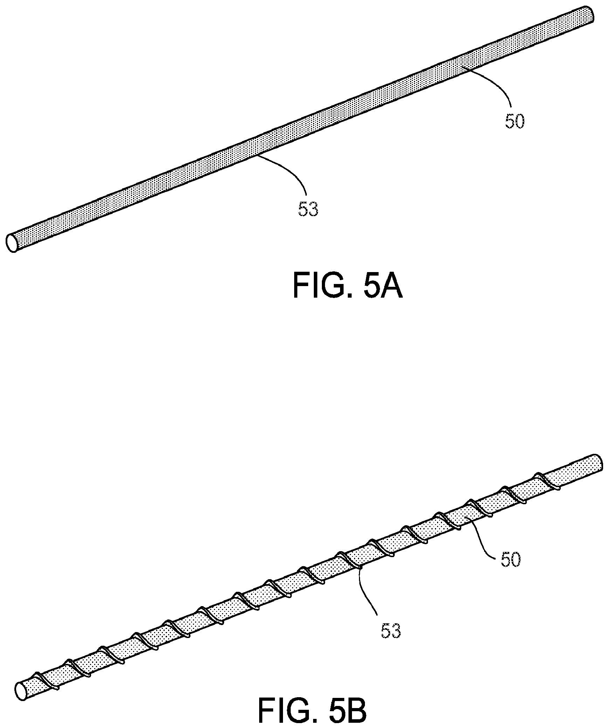

With reference now to FIGS. 5A-5B, a spiral configuration may be used. FIG. 5A illustrates a first elongate element 50 having a second elongate element 53 (e.g., a polymer filament) spirally wrapped upon first elongate element 50 in a relatively tight pitch and sintered together with it. FIG. 5B illustrates a first elongate element 50 having a second elongate element 53 spirally wrapped upon first elongate element 50 in a relatively loose pitch and sintered together with it.

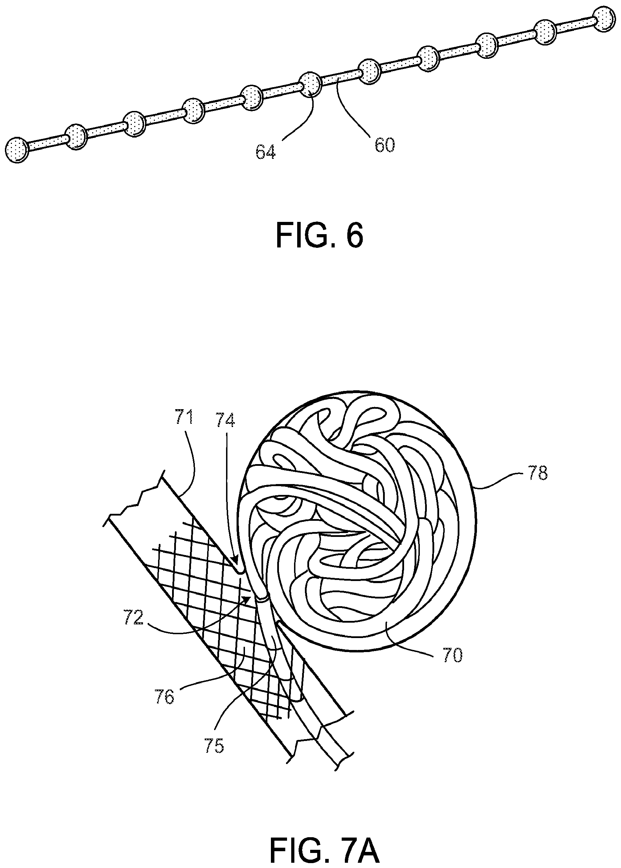

Turning now to FIG. 6, one or a plurality of beads 64 on an elongate element 60 may also be used. In a preferred embodiment, beads 64 are polymer beads that are "painted on" as known in the art. In accordance with an embodiment of the present invention, one or a plurality of beads 64 are coated with a polymer containing a radio-opaque or echogenic additive.

More broadly, any portion of the elongate element (or any structural modification made thereto) may comprise a radio-opaque or echogenic element that enhances imaging or detection of the elongate element during and following implantation, such as disclosed in U.S. Publication No. 2004/0024448. Preferred radio-opaque markers may be comprised of one or more of tungsten, gold, platinum and the like. In applications of the present invention wherein the elongate element may be removable following implantation, radio-opaque elements may be particularly advantageous.

Other example embodiments may be rendered hydrophilic and then soaked in radio-opaque contrast prior to delivery. Soaking in contrast will facilitate a temporarily radiographically visible device. Once the contrast washes out, the device will become radiographically transparent. Additionally, in embodiments comprising ePTFE, un-wet ePTFE may be sufficiently echogenic during delivery and eventually wet out and become transparent after delivery. Similarly, as previously described, the second reagent can be combined with a radio-opaque contrast agent.

In general, any modification to adjust a dimensional characteristic of the elongate element may be suitable for use in connection with the disclosed embodiments.

FIGS. 7A-7C provide example elongate element 70 deployment systems. In general, a catheter 75 can be routed within a bodily vessel 71 to the site of an aneurysm neck 74. The elongate element 70 can be deployed from catheter 75 into an aneurysm sac 78 or other type of occlusion space. As explained below, a stabilization member, such as a stent or a balloon, can be used to maintain the catheter 75 in the deployment position and prevent any portion of elongate element 70 from escaping the aneurysmal compartment and protruding into the host vessel lumen. After deployment of the elongate element 70, the stabilization member may be removed, or may remain in the vessel 71. The deployment configuration may be as shown in the FIGS. 7A-7C, or by any combination of the features and devices provided therein.

As shown in FIG. 7A, the elongate element 70 of the present invention may be delivered from within a vessel 71, over a catheter 75 or out from within (i.e., through) a catheter 75. The catheter 75 may be a stent-crossing catheter, as shown in FIG. 7A, configured to cross a stent 76, for example, to occlude an aneurysm located off of the vessel 71. The elongate element 70 may be deployed while the neck of the aneurysm 74 is supported by stent 76. Any of a variety of types of stents can be used, such as a braided or a lantern type. The elongate element 70 may exit from a distal tip 72 of the catheter 75 and enter the aneurysm sac 78 via an aneurysm neck 74.

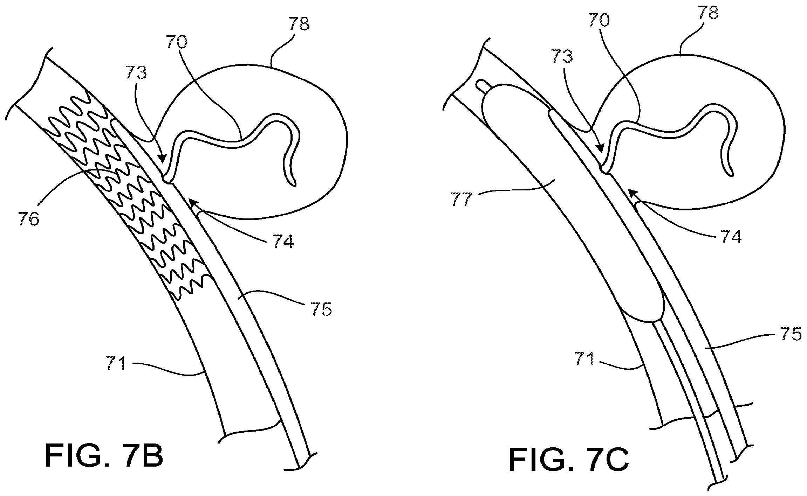

As shown in FIG. 7B, the catheter 75 may be positioned between the wall of vessel 71 and stent 76, rather than being routed through the stent 76. The elongate element 70 may exit from the catheter 75 via a side-wall aperture 73 of the catheter 75, rather than at the tip or distal end of the catheter 75. The catheter 75 may include an internal ramp structure (not shown) so as to gradually direct the elongate element 70 to exit from catheter 75 in a generally radial direction via the side-wall aperture 73. Radio-opaque markers can be located in various locations on the catheter 75-including on the interior ramp-and on the elongate element 70 to aid with positioning the catheter aperture 72/73 in relation to the aneurysm neck 74.

As shown in FIG. 7C, the stabilization member can alternately be a balloon 77 instead of a stent. The balloon 77 can be delivered to the area of the aneurysm by a catheter and can hold catheter 75 against the wall of vessel 71. Generally, balloons may be a preferred type of stabilization member for aneurysms with small necks, whereas stents may be preferred for aneurysms with larger necks.

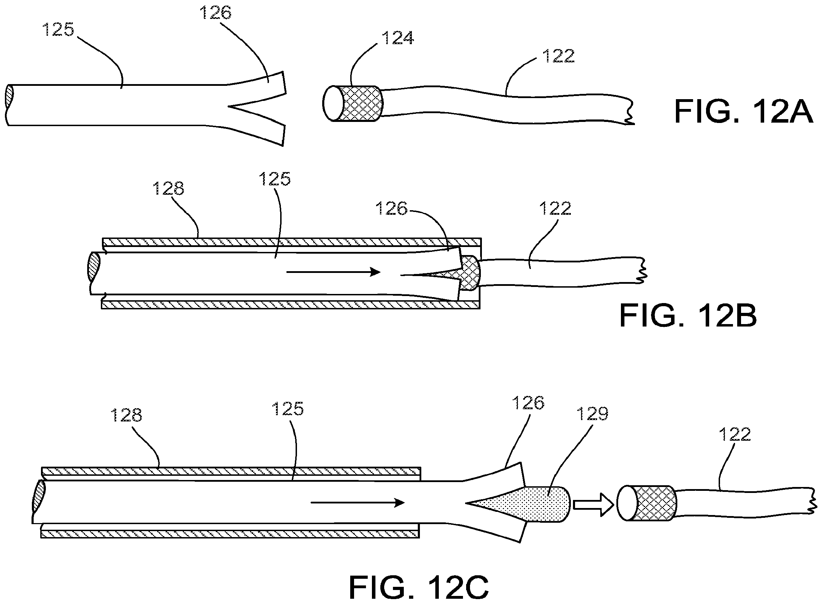

FIGS. 12A-12C, depict first example embodiments of an elongate element conveyance system. In general, the embodiment performs by using a gripping tube to push the elongate element through a catheter lumen and into the occlusion space.

FIG. 12A shows (on the right) a portion of an elongate element 122 including an optional radio-opaque marker 124 at the proximal end of elongate element 122. Also shown (on the left) is a portion of a tube 125 that can have a split-end 126 resembling a "duckbill." Tube 125 can be, for example, a metal or polymeric tube that is extruded, drawn, molded, or spirally formed. In a preferred embodiment, tube 125 can be comprised of nitinol material. The split-end 126 can be biased to normally reside in an open or expanded state as depicted in FIG. 12A. In that expanded state, the inner dimensions of split-end 126 are larger than the outer dimensions of the elongate element 122 or the radio-opaque marker 124-resulting in an interference fit between the tube 125 and the elongate element 122.

FIG. 12B shows the conveyance system including a catheter 128 to illustrate the configuration of the system during the conveyance of an elongate element. For example. tube 125 and elongate element 122 can be disposed within a lumen of the catheter 128 (shown in cross-section). The size of the inner diameter of the lumen can cause the split-end 126 of tube 125 to pinch and hold the elongate element 122. That is, the lumen's inner diameter can be smaller than an outer dimension of split-end 126. Therefore, while the tube 125 is disposed within the lumen, the lumen wall can exert compressive forces on the split-end 126 to cause it to be reduced in size from its expanded state. In the reduced size state within the lumen, the inner diameter of the split-end 126 pinches and couples with the elongate member 122. The coupled combination of the tube 125 and the elongate element 122 can be pushed through the lumen of catheter 128 (e.g., in the direction of the arrow in FIG. 12B) to thereby convey the elongate element 122 towards the occlusion site, and allow the elongate element 122 to be retrieved by reversing direction of tube 125.

FIG. 12C shows the conveyance system with the split-end 126 of tube 125 having exited from the distal end of the lumen of catheter 128. In this configuration the split-end 126, having been freed from the compressive forces previously exerted by the lumen wall, can return to its expanded state. Therefore, the split-end 126 no longer pinches elongate element 122 and elongate element 122 can decouple from tube 125. In order to assist in the decoupling, a stylet 129 can be optionally used to force the elongate element 122 away from the tube 125 an into the occlusion space.

Some embodiments of the occlusion device system provided herein can treat multiple endovascular occlusion sites during a single treatment session. For example, the delivery catheter can be positioned at a first endovascular location and can deliver one or more segments of elongate element material to occlude a first occlusion space (e.g., a first aneurysm). Then, without removing the delivery catheter from the patient, the catheter can be repositioned to a second location and can deliver one or more elongate element material segments to occlude a second occlusion space (e.g., a second aneurysm). If desired, a third space can be treated (again, without removing the delivery catheter from the patient), and so on.

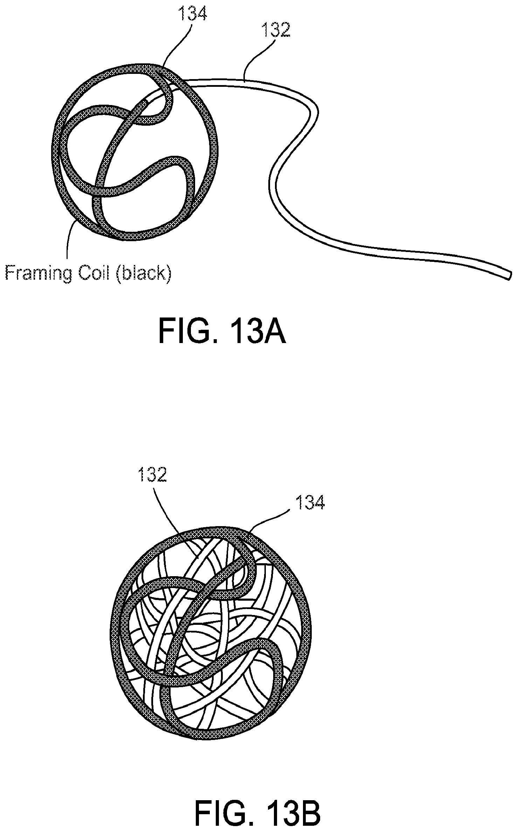

FIGS. 13A-13B depict additional example embodiments of an occlusion device. This embodiment includes a combination of two types of elongate elements: (i) elongate element 132 that can be an occluder as described above (e.g., in reference to FIGS. 1-7C) and (ii) another elongate element 134 that is stiffer than embodiments of the elongate element described previously. In some cases, elongate element 134 is a wire. The elongate element 134 can be a metallic or polymeric material and can have a solid-core or be a tube. In a preferred embodiment, the elongate element 134 is a solid-core nitinol wire that can exhibit shape-memory characteristics. A trailing-end of the elongate element 134 can be fixedly attached to a leading-end of the elongate element 132.

The elongate elements can be deployed to an occlusion site in the following manner. The elongate element 134 can be deployed to the occlusion site as the leading-end of the combined elongate elements. As the elongate element 134 exits the delivery catheter (not shown), the elongate element 134 can create a generally spherical outer frame as depicted in FIG. 13A. The elongate element 134 can be predisposed to such a shape from having shape-memory characteristics, or it may become so shaped simply from seeking the outer boundaries of the space (e.g., an aneurysm sac) by virtue of being the first occluder element to enter the space.

As the elongate element 132 exits the catheter, it can begin to fill the volume defined within the elongate element 134 outer framework. As shown in FIG. 13B, the elongate element 132 can be compacted into the framework to complete the filling of the occlusion space. In some embodiments, the elongate element 132 can have regions of varying geometry or modulus of elasticity to assist in filling the space (as described above regarding FIG. 1).

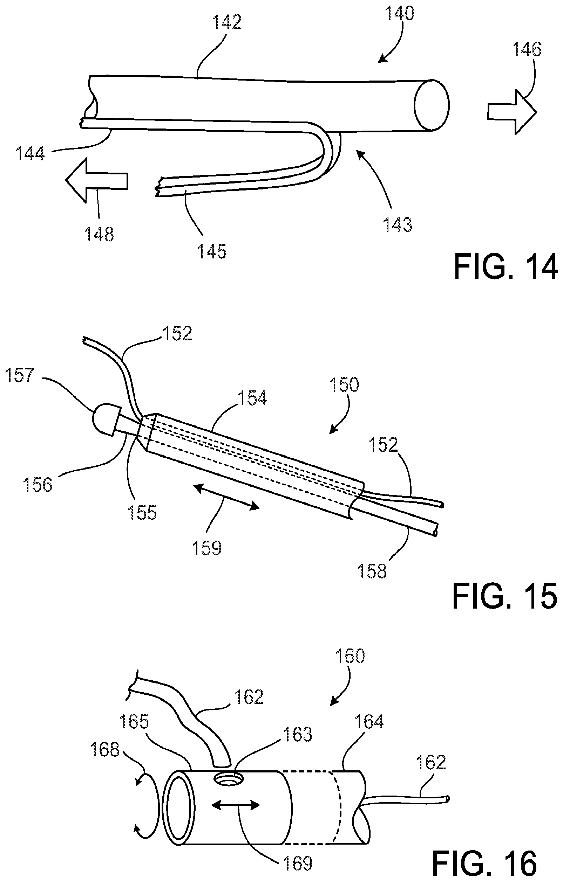

FIG. 14 illustrates additional example embodiments of a delivery system 140. Delivery system 140 conveys elongate element 142 to a delivery site with a carrier 143. In general, delivery system 140 includes an elongate element 142, a carrier 143, and a catheter (not shown). The elongate element 142 can be as described above, for example in reference to FIG. 1. The catheter can have at least two lumens. As will be described in more detail below, carrier 143 may include a conveying portion 144 that delivers the elongate element 142 through a first lumen of the catheter, and a returning portion 144 that returns from a distal end of the catheter to a proximal end of the catheter through a second lumen of the catheter. That is, the first lumen can be used for the delivery of elongate element 142, where elongate element 142 is in physical contact with the conveying portion 144 of carrier 143. The second lumen can be used for the return of the returning portion 145 of carrier 143.

In some embodiments, the carrier 143 can be a looped material. The conveying portion 144 can be in contact with the elongate element 142. In some embodiments, the elongate element 142 can be tacked to the conveying portion 144 of the carrier 143 to assist the elongate element 142 to move through the catheter's first lumen in conjunction with the conveying portion 144. The movement of the carrier 143 can be induced by the application of a tensile force acting in the direction of arrow 148. That is, the returning portion 145 can be urged in the direction of arrow 148 to cause the entire length of carrier 143 to move with respect to the catheter, and to convey the elongate member 142 into the occlusion space.

When a portion of the conveying portion 144 of the carrier and the elongate element 142 reach the distal tip of the catheter, the carrier loops back into the second lumen of the catheter, separating from the elongate element 142, and the elongate element continues on into the delivery site in the direction of arrow 146. Carrier 143 may make about a 180-degree turn and reenter the catheter via the second lumen in the direction of arrow 148.

FIGS. 15-19D illustrate example embodiments of an occlusion device system including various types of cutting mechanisms that can sever the elongate element to aid in the delivery of the proper length of elongate element to fill the occlusion space. In general, the cutting mechanisms are located at or near the distal tip of a catheter.

FIG. 15 provides an example cutting mechanism 150. Elongate element 152 can be transmitted through a lumen of catheter body 154. Also associated with the catheter body 154 is a cutter actuation member 158 that can be axially movable with respect to the catheter body 154 (as depicted by arrow 159). The end of the cutter actuation member 158 can comprise a nose 157. Proximally adjacent to the nose 157 can be a ramp 156. The distal end of catheter body 154 can include a cutting edge 155. To sever the elongate element 152, the cutter actuation member 158 can be moved proximally with respect to the catheter body 154 to move the nose 157 towards the cutting edge 155. The ramp 156 can cause the elongate element 152 to move radially to become adjacent to the cutting edge 155. As the cutter actuation member 158 continues to be moved proximally, the elongate element 152 can become captured between the cutting edge 155 and the rear planar surface of the nose 157. Further proximal movement of the cutter actuation member 158 can then cause the elongate element 152 to be severed.

FIG. 16 depicts an example cutting mechanism 160 disposed on a catheter 164 with a side-wall aperture 163. Elongate element 162 can be transmitted through a lumen of the catheter body 164 and exit through the side-wall aperture 163. The side-wall aperture 163 comprises an opening through a tip portion 165 and a similar opening (not shown) in the catheter body 164. The tip portion 165 can be movable with respect to the catheter body 164. In one embodiment, the tip portion 165 can be movable in an axial direction as represented by arrow 169. In another embodiment, the tip portion 165 can be movable in a rotary manner as depicted by arrow 168. The motion of tip portion 165 with respect to the catheter body 164 can cause the elongate element 162 to be sheared at the side-wall aperture 163. In some implementations, the catheter body 164 is movable with respect to the tip portion 165.

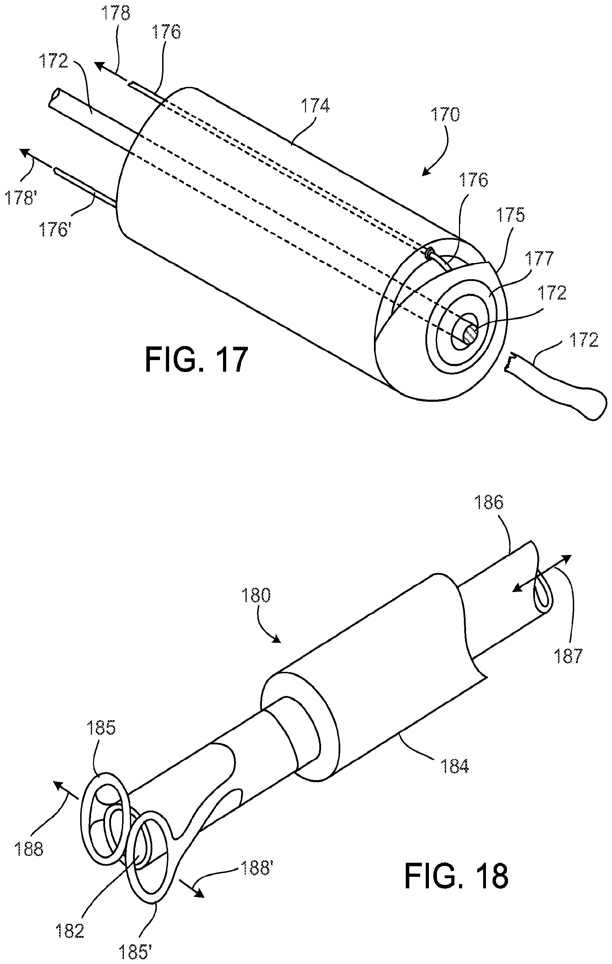

FIG. 17 depicts another example cutting mechanism 170. The elongate element 172 can be transmitted through a lumen in the catheter body 174. A pivotable cutter 175 can be disposed near the distal tip of the catheter body 174. The pivotable cutter 175 can be adjacent to a spherical gimbal 177 of the catheter body 175, or another member that can provide a cutting surface corresponding with the pivotable cutter 175. The pivotable cutter 175 can be actuated using cutter actuator members 176 and 176' that can be attached to opposite sides of the pivotable cutter 175. For example, the cutter actuator members 176 and 176' can be wires. To pivot the pivotable cutter 175, the cutter actuator members 176 or 176' can be individually pulled in the directions represented by the arrows 178 and 178' respectively. That is, one cutter actuator member (176 or 176') at a time can be pulled to pivot the pivotable cutter 175 with respect to the spherical gimbal 177. As the pivotable cutter 175 is actuated to cause it to pivot, the apertures of the pivotable cutter 175 and the spherical gimbal 177 become misaligned and the elongate element 172 can be thereby severed. The pivotable cutter 175 can thereafter be pivoted back to a position wherein the apertures are in alignment. In this manner the cutting mechanism 170 can be resettable. That is, after delivering and cutting a first segment of elongate element 172 at an occlusion space, one or more subsequent segments of elongate element can be delivered and cut, at the same or a different occlusion space, without having to remove the cutting mechanism 170 from the vasculature of the patient.

FIG. 18 depicts another example cutting mechanism 180. This embodiment includes an inner catheter body 186 disposed within an outer catheter body 184. The catheter bodies 184 and 186 can be axially movable with respect to each other as depicted by arrow 187. The elongate element 182 can be transmitted through a lumen of the inner catheter body 186. Cutters 185 and 185' can be attached to inner catheter body 186, and can include sharpened cutting surfaces at their distal ends. In some embodiments a single cutter can be used. The cutters 185 and 185' can be biased to deflect radially away from the axis of the inner catheter 186 in the directions depicted by arrows 188 and 188'. That configuration, as shown, is a cutting configuration. In a normal operating configuration, the inner catheter 186 can be disposed within the outer catheter 184 such that the cutters are displaced radially inward and contained between the catheter bodies 184 and 186 in a position that allows the elongate element to be freely transmitted from the distal tip of the inner catheter 186. That is, the inner wall of the outer catheter 184 can contact the cutters 185 and 185' to urge the cutters 185 and 185' to a position adjacent to the outer wall of the inner catheter body 186. In that position, the distal sharpened cutting surfaces of cutters 185 and 185' do not inhibit the elongate element 182 from exiting the inner catheter body 186. The elongate element 182 can be severed by axially moving the catheter bodies 184 and 186 with respect to each other such that the inner catheter body 186 extends distally from the outer catheter body 184, thereby freeing the cutters 185 and 185' to displace radially as shown in FIG. 18. As the cutters 185 and 185' displace to the cutting configuration, the sharpened cutting surfaces can sever the elongate element 182. The cutters 185 and 185' can thereafter be repositioned to their normal operating configuration. In this manner the cutting mechanism 180 can be resettable. That is, after delivering and cutting a first segment of elongate element 182 at an occlusion space, one or more subsequent segments of elongate element can be delivered and cut, at the same or a different occlusion space, without having to remove the cutting mechanism 180 from the vasculature of the patient.

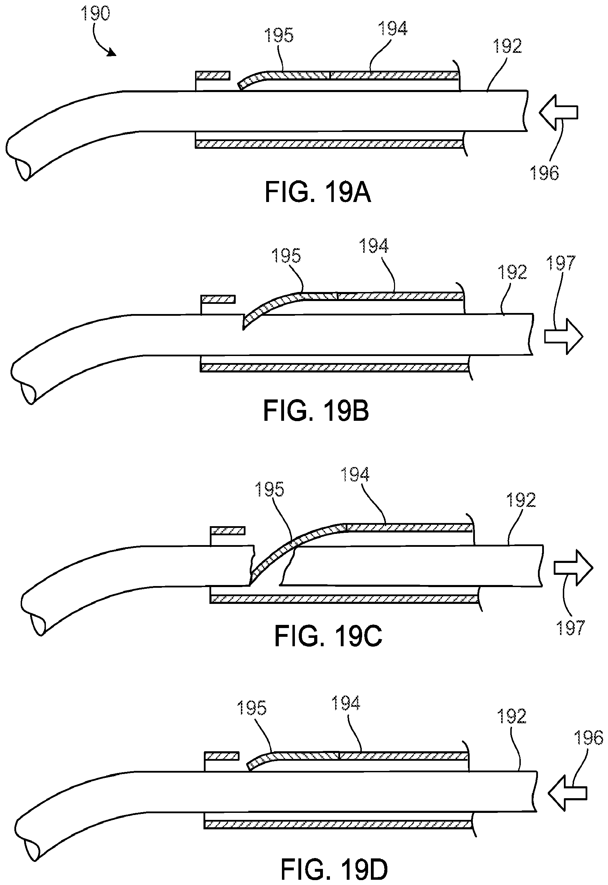

FIGS. 19A-19D depict another embodiment of a cutting mechanism 190. The elongate element 192 can be transmitted through a lumen of the catheter 194. A flap cutter 195 with a sharpened distal end can be attached to the catheter 194 near its distal end. The flap cutter 195 can be biased to a deflected position as depicted in FIG. 19C. However, in the normal operating configuration, the flap cutter 195 can gently rest in contact with the elongate element 192 as depicted in FIG. 19A. In the normal operating configuration, the elongate element 192 is moved in the direction of arrow 196 so as to deliver elongate element 192 to an occlusion space. In that case, the flap cutter 195 allows the elongate element 192 to pass under it relatively uninhibited. To initiate the severing of elongate element 192, the elongate element 192 can be pulled in a proximal direction as depicted by arrow 197. In that case, the sharpened distal end of the flap cutter 195 will begin to bite into the elongate element 192 as shown in FIG. 19B. As the pulling of elongate element 192 in the direction of arrow 197 continues, the flap cutter 195 can sever the entire diameter of the elongate element 192 as shown in FIG. 19C. After the cutting operation, the transmission of elongate element 192 can be resumed by once again moving the elongate element 192 in the direction of arrow 196 as shown in FIG. 19D. That is, after delivering and cutting a first segment of elongate element 192 at an occlusion space, one or more subsequent segments of elongate element can be delivered and cut, at the same or a different occlusion space, without having to remove the cutting mechanism 190 from the vasculature of the patient.

The elongate element of the present invention may also be surgically implanted and activated in situ. Further, the elongate element may be incorporated into an implantable prosthesis.

Hydraulic or mechanical approaches may be used to deliver the elongate element(s), whether it is delivered over or out from within a catheter. According to the invention, hydraulic agents can be used to propel the elongate element through a lumen of a delivery catheter. In the case of hydraulic approaches, various liquids such as saline, radio-opaque contrast or a block copolymer, as described herein, are preferably although not exclusively used as the propelling agent. Hydraulic delivery can be generally performed by pressurizing the proximal end of a lumen containing an elongate element. The pressure can cause the elongate element to be propelled distally, and into the occlusion space. This delivery technique is, according to the invention, enhanced by sealing the interface between the elongate element and the walls of the lumen. Various elongate element design features can provide an enhanced seal between the elongate element and the walls of the lumen. According to the invention, the auger configuration illustrated in FIGS. 4A and 4B, the spiral wraps of FIGS. 5A and 5B, and the beaded configuration of FIG. 6 provide such an enhanced seal. In addition to pressure-induced propulsion, in some embodiments the elongate element can be transmitted through the lumen of a delivery catheter by being carried with the momentum of the flow of a hydraulic agent. Mechanical delivery approaches can include, for example, applying a mechanical force to the elongate element to push the elongate element through the lumen of a delivery catheter. For example, in some embodiments a stylet can be used to push the elongate element through the lumen and into the occlusion space. In some embodiments, a combination of hydraulic and mechanical delivery techniques can be used.

In example embodiments, the elongate element is imbibed with a first reagent and a second reagent is used to hydraulically deliver the elongate element. A resulting gel or other product may, or in other embodiments, need not be, isolated within the elongate element.

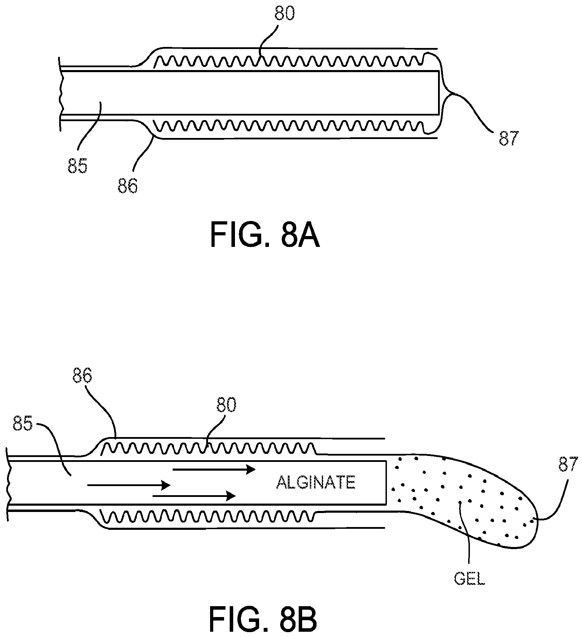

In some embodiments, and with reference to FIGS. 8A-8B, elongate element 80 is tubular and is imbibed with a polyvalent compound as described above, for example calcium or a calcium chloride compound. Elongate element 80 is delivered over a catheter 85 from under a sheath 86, and such delivery can be hydraulically powered by the infusion or flow of saline through catheter 85 in the direction of a sealed end 87 of elongate element 80.

In some embodiments, and with reference to FIGS. 8A-8B, elongate element 80 is tubular and is imbibed with a polyvalent compound as described above, for example calcium or a calcium chloride compound. Elongate element 80 is delivered over a catheter 85 from under a sheath 86, and such delivery can be hydraulically powered by the infusion or flow of a block copolymer through catheter 85 in the direction of a sealed end 87 of elongate element 80.

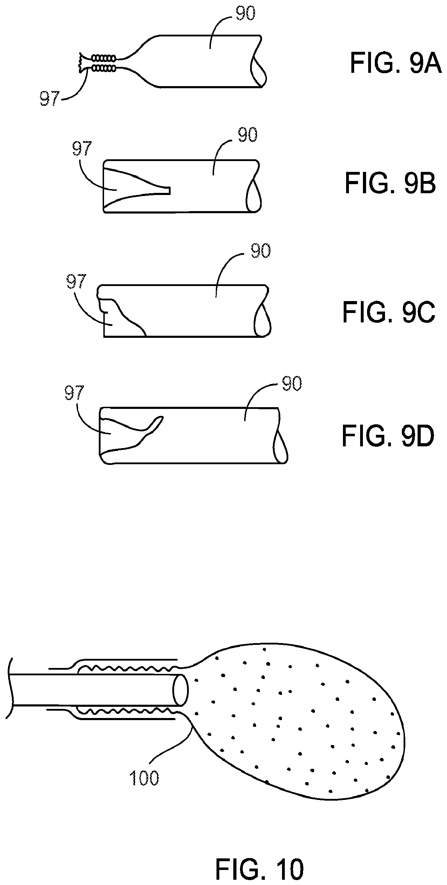

The elongate element may be sealed at one end in a variety of manners, as shown in FIGS. 9A-9D. With reference to FIG. 9A, an elastomer may be used to restrict an end 97 of an elongate element 90. As shown in FIG. 9B, an end 97 of an elongate element 90 may be everted within itself. Turning to FIG. 9C, an end 97 of an elongate element 90 may comprise a flap folded back on itself. Finally, and with reference to FIG. 9D, an end 97 of an elongate element 90 may comprise a variation of everted and kinked portions. In general, any valve-like configuration that seals or otherwise kinks the elongate element is suitable for use in connection with some embodiments.

As noted above, example embodiments comprise a distensible elongate element 100, which may find particular utility in applications where a larger version occlusive device is required, as shown in FIG. 10.

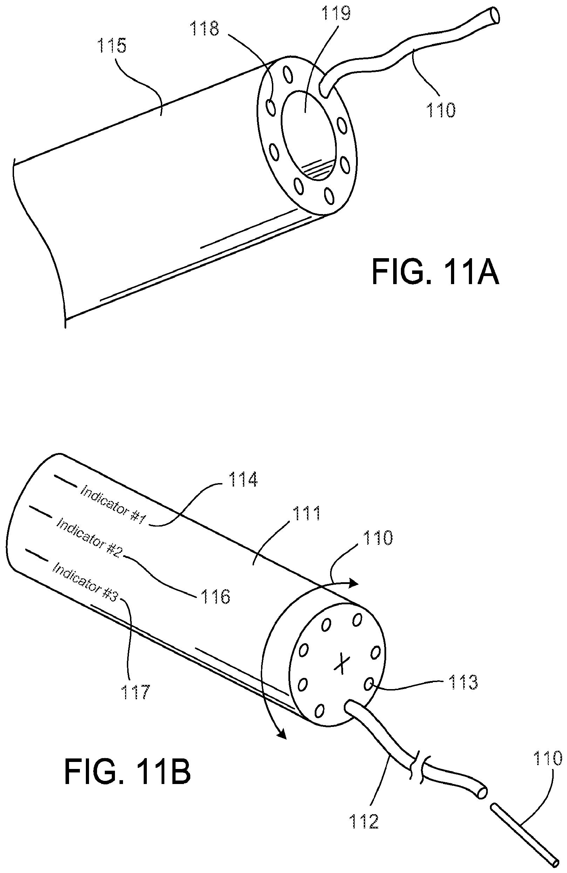

Yet another possible delivery system is illustrated in FIG. 11A. Multiple elongate element 110 segments may be housed in multiple lumens 118 within at least the distal end of a catheter 115. In some embodiments, the multiple lumens 118 can extend approximately the entire length of the catheter 115. The delivery system can be provided to a clinician operator with the multiple lumens 118 pre-filled with elongate element 110 segments. The segments of elongate element 110 can be deployed individually as necessary during a procedure. A variety of lengths of elongate element 110 segments can be housed in the multiple lumens 118 (e.g., 1", 2", 3", 4", 6", 8", 1', 1.5', 2', 3', 4', 5', 6'). In this way, the catheter 115 may forgo the need for a cutting mechanism. For example, at the start of an occlusion procedure the clinician operator may choose to deploy a long segment of elongate element 110 to fill most of the space being occluded. The entire segment can be deployed into the space. As the space becomes close to being filled, the clinician can choose to deploy a shorter segment of elongate element 110 that is the appropriate length to approximately fill the remaining space. In some cases, the multi-lumen delivery system can be used to treat multiple occlusion spaces without the need to refill the delivery system with elongate element 110. The deployment of the elongate element 110 segments from the multiple lumens 118 of catheter 115 can be performed using any of the techniques describe herein, and preferably using hydraulic and/or mechanical techniques.

In some example embodiments, central lumen 119 is used for delivery of the guidewire, yet central lumen 119 may additionally or alternatively be used for delivery of an elongate element or alginate solution. In addition, tube delivery may be combined in this delivery system, including the possibility of deploying a tube by filling it with an elongate element.

The operational concept, as provided in reference to FIG. 11A, of using multiple lumens to dispense individual segments of elongate element, can also be applied in an embodiment illustrated, for example, in FIG. 11B, where multiple lumens 113 are disposed in dispenser 111. In some embodiments, dispenser 111 may be a portion of a multi-lumen catheter that operates as an elongate element dispenser at the proximal end of the system. This embodiment can include a distal catheter 112 including a distal-end delivery lumen for the delivery of elongate element segments 110 from the distal catheter 112 to an occlusion space. In some embodiments, the distal catheter 112 can include more than one lumen. The example embodiment of FIG. 11B can be particularly suited to accessing occlusion spaces in tight areas such as in, but not limited to, neurovascular settings.

The distal-end delivery lumen of the distal catheter 112 can be selectively coupled with individual dispensing lumens of the multiple lumens 113 to dispense segments of elongate element from the dispenser 111. For example, in some embodiments the dispenser 111 can be selectively rotated about its longitudinal axis in the direction depicted by arrow 110. As the dispenser 111 is rotated, the distal catheter 112 remains stationary with respect to the dispenser 111. In this manner, any one of the multiple lumens 113 can become individually selectively aligned and in fluid communication with the distal-end delivery lumen of the distal catheter 112, to be positioned to dispense its segment of elongate element to the distal catheter 112. In other embodiments, the proximal multi-lumen dispenser portion can have a non-cylindrical configuration. For example, the dispenser 111 can have a linear configuration such that the axes of the multiple dispensing lumens are arranged on a common plane. In that case, the dispenser portion would be linearly movable to individually align its dispensing lumens with the distal catheter. In another embodiment, the multi-lumen dispenser portion can have a rectangular configuration. In that case, the dispenser portion would be movable in two planes (e.g., x-y axes) to align its dispensing lumens with the distal catheter. In addition, other embodiments can have other variations of dispensing lumen configurations, such as ovals, helixes, and polygons.

The individual lumens 113 of the dispenser 111 can contain and dispense segments of elongate elements with disparate lengths (e.g., 1", 2", 3", 4", 6", 8", 10", 1', 2', 3', or greater). The dispenser 111 can include one or more of indicators 114, 116, and 117 that are visible to the clinician operator. These indicators 114, 116, and 117 correspond with individual dispensing lumens of the multiple lumens 113. By knowing what particular segments of elongate material are contained within the individual dispensing lumens, the indicators 114, 116, and 117 can be correlated with the lengths of the elongate element segments housed within the dispensing lumens. In this manner the clinician operator, knowing which individual dispensing lumen is coupled with the distal catheter 112, can be apprised of what segment length of elongate element the multi-lumen portion is configured to dispense, in some implementations. The indicators 114, 116, and 117 can be used to identify various properties pertaining to an individual dispensing lumen. For example, among other things, the indicators 114, 116, and 117 can identify a dispensing lumen number, a segment length of elongate element contained in a dispensing lumen, or a volume of occlusion space that can be filled by the segment of elongate element contained in a dispensing lumen. In some embodiments, at least the proximal end of the dispenser 111 is positioned outside of the body of the patient being treated. In some embodiments, the junction of the dispenser 111 and the distal catheter 112 is positioned outside of the body of the patient being treated.

In certain embodiments of an endovascular occlusion system, the elongate element may be delivered along with a balloon, for example with a dual-lumen catheter. In a preferred embodiment, a balloon is delivered along with the elongate element in its first configuration to a treatment site, whereupon both are deployed and the balloon remains deployed where occlusion is not desired until the elongate element has assumed its second, activated or reacted (e.g., cross-linked) configuration where occlusion is desired. The balloon may then be retrieved.

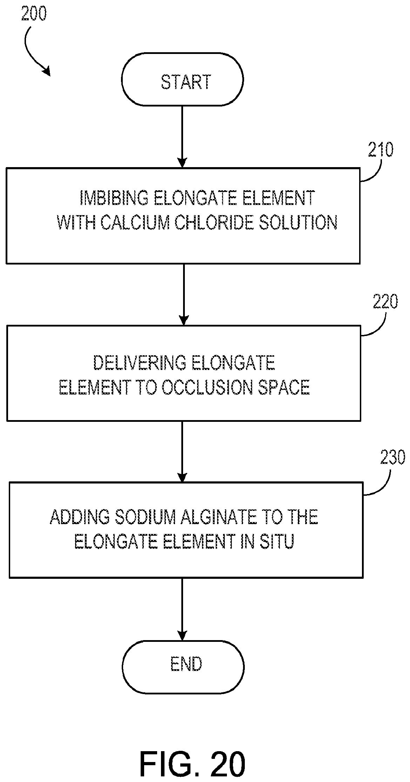

FIG. 20 depicts an example embodiment of a method 200 for endovascular occlusion. At operation 210 an elongate element can be imbibed with a calcium chloride solution. This can be achieved, for example, by soaking a porous elongate element, such as an elongate element comprised of ePTFE, in a calcium chloride solution. At operation 220, the imbibed elongate element can be delivered to an occlusion space. The delivery systems provided herein, such as a delivery catheter, can preferably be used for this action. At operation 230, a sodium alginate can be added to the imbibed elongate element in situ. That is, after the imbibed elongate element has been delivered to the occlusion space, the sodium alginate can be delivered to the occlusion space to come in contact with the imbibed elongate element. The combination of the elongate element imbibed with calcium chloride and the sodium alginate can form a gel and can cause the elongate element to expand to fill the occlusion space. In an alternative embodiment, the elongate element can be imbibed with the sodium alginate and the calcium chloride can be added in situ.

In some embodiments where the elongate element comprises a tube, a coil or a plurality of coils may be delivered into the tube, and delivery of the coil or coils may cause the tube to be deployed into an occlusion site.

Related methods are also contemplated herein and may be used in connection with occlusion of aneurysms of all sizes (e.g., intracranial and aortic aneurysms), vessels of all sizes, venous and arterial, sequestration of diseased tissue, and endoleaks, to name just a few applications. The present invention may also be used in connection with the unwanted retrograde infusion associated with the delivery and deployment of endovascular devices.

It will be apparent to those skilled in the art that various modifications and variations can be made in the present invention without departing from the scope of the invention. Thus, it is intended that the present invention cover the modifications and variations of this invention provided they come within the scope of the appended claims.

By way of non-limiting example, while the present invention has been described primarily with reference to the cardiovascular system, those skilled in the art will appreciate that the invention is not so limited, but may have utility more broadly wherever filling or occlusion is required. The present invention may also have applicability as an implantable/removable drug-elution reservoir.

## Claims

1. A system for occluding, comprising:
a catheter (70) comprising a lumen,
an elongate element (70) located within said lumen,
wherein the elongate element is configured to be delivered to a site for occlusion upon hydraulic flow through the lumen of the catheter,
wherein the elongate element is porous and
wherein the elongate element comprises a first configuration suitable for delivery from the catheter, and a second configuration suitable for occlusion following delivery to the site for occlusion, wherein the elongate element has no bias such that the second configuration is random,
**characterised in that** said elongate element (70) comprises features to provide an enhanced seal between the elongate element and the lumen selected from an auger configuration (42), a spiral configuration (53) and one or a plurality of beads (64) and **in that** the elongate element (70) is imbibed with at least one of a therapeutic composition, swellable agent, bioactive agent, drug, or compound.

2. The occlusion system of claim 1, further comprising a cutter (150, 160, 170, 180, 195) adapted to cut the elongate element, the cutter being resettable to a cut-ready state without withdrawing the occlusion device from a patient, wherein the elongate element (162) is delivered through a lumen of a catheter (164), the lumen including a first aperture (163) in a side wall of the catheter, and wherein the cutter (160) comprises a tube portion (165) concentric with a distal portion of the catheter (164) and includes a second aperture in a side wall of the tube portion.

3. The occlusion system of claim 1, wherein the elongate element is imbibed with a first reagent and the system comprises a second reagent configured to hydraulically deliver the elongate element.

4. The occlusion system of claim 1, wherein the elongate element (80) is tubular and comprises a sealed end (87), wherein the elongate element is imbibed with a polyvalent compound.

5. The occlusion system of claim 4, wherein the elongate element is configured to be delivered over a catheter (85) and such delivery is hydraulically powered by the infusion or flow of saline through the catheter (85) in the direction of the sealed end (87) of the elongate element (80).

6. The occlusion system of claim 4, wherein the elongate element is configured to be delivered over a catheter (85) and such delivery is hydraulically powered by the infusion or flow of a block copolymer through the catheter (85) in the direction of the sealed end (87) of the elongate element (80).

7. The occlusion system of claim 1, further comprising multiple elongate elements (110) housed within multiple lumens (118) within at least the distal end of the catheter (115).

8. The occlusion system of claim 7, wherein the multiple elongate elements have a variety of lengths and can be deployed individually.

## Patentansprüche

1. Okklusionssystem, umfassend:
einen Katheter (70), der ein Lumen umfasst,
ein längliches Element (70), das sich innerhalb des Lumens befindet,
wobei das längliche Element konfiguriert ist, um bei hydraulischem Fluss durch das Lumen des Katheters zu einer Stelle zur Okklusion zugeführt zu werden,
wobei das längliche Element porös ist
und
wobei das längliche Element eine erste Konfiguration, die für eine Zuführung aus dem Katheter geeignet ist, und eine zweite Konfiguration, die für eine Okklusion nach Zuführung zu der Stelle zur Okklusion geeignet ist, umfasst, wobei das längliche Element keine Vorspannung aufweist, sodass die zweite Konfiguration zufällig ist, **dadurch gekennzeichnet, dass** das längliche Element (70) Merkmale umfasst, um eine verbesserte Abdichtung zwischen dem länglichen Element und dem Lumen bereitzustellen, ausgewählt aus einer Schneckenkonfiguration (42), einer Spiralkonfiguration (53) und einem oder einer Vielzahl von Wülsten (64), und dass das längliche Element (70) mit mindestens einem von einer therapeutischen Zusammensetzung, einem quellbaren Agens, einem bioaktiven Agens, einem Arzneimittel oder einer Verbindung getränkt ist.

2. Okklusionssystem nach Anspruch 1, ferner umfassend ein Schneidemesser (150, 160, 170, 180, 195), das zum Schneiden des länglichen Elements angepasst ist, wobei das Schneidemesser in einen schneidbereiten Zustand rückstellbar ist, ohne die Okklusionsvorrichtung aus einem Patienten zurückzuziehen, wobei das längliche Element (162) durch ein Lumen eines Katheters (164) zugeführt wird, wobei das Lumen eine erste Öffnung (163) in einer Seitenwand des Katheters beinhaltet, und wobei das Schneidemesser (160) einen Röhrenabschnitt (165) umfasst, der mit einem distalen Abschnitt des Katheters (164) konzentrisch ist und eine zweite Öffnung in einer Seitenwand des Röhrenabschnitts beinhaltet.

3. Okklusionssystem nach Anspruch 1, wobei das längliche Element mit einem ersten Reagens getränkt ist und das System ein zweites Reagens umfasst, das konfiguriert ist, um das längliche Element hydraulisch zuzuführen.

4. Okklusionssystem nach Anspruch 1, wobei das längliche Element (80) röhrenförmig ist und ein abgedichtetes Ende (87) umfasst, wobei das längliche Element mit einer polyvalenten Verbindung getränkt ist.

5. Okklusionssystem nach Anspruch 4, wobei das längliche Element konfiguriert ist, um über einen Katheter (85) zugeführt zu werden, und eine solche Zuführung durch die Infusion oder den Fluss von Salzlösung durch den Katheter (85) in die Richtung des abgedichteten Endes (87) des länglichen Elements (80) hydraulisch angetrieben wird.

6. Okklusionssystem nach Anspruch 4, wobei das längliche Element konfiguriert ist, um über einen Katheter (85) abgegeben zu werden, und eine solche Abgabe durch die Infusion oder den Fluss eines Blockcopolymers durch den Katheter (85) in die Richtung des abgedichteten Endes (87) des länglichen Elements (80) hydraulisch angetrieben wird.

7. Okklusionssystem nach Anspruch 1, ferner umfassend mehrere längliche Elemente (110), die innerhalb mehrerer Lumen (118) innerhalb mindestens des distalen Endes des Katheters (115) untergebracht sind.

8. Okklusionssystem nach Anspruch 7, wobei die mehreren länglichen Elemente eine Vielzahl von Längen haben und einzeln eingesetzt werden können.

## Revendications

1. Système pour occlusion, comprenant :
un cathéter (70) comprenant une lumière,
un élément allongé (70) situé à l'intérieur de ladite lumière,
dans lequel l'élément allongé est configuré pour être administré à un site d'occlusion lors d'un débit hydraulique à travers la lumière du cathéter,
dans lequel l'élément allongé est poreux et
dans lequel l'élément allongé comprend une première configuration permettant l'administration à partir du cathéter, et une deuxième configuration permettant l'occlusion à la suite de l'administration au site d'occlusion, dans lequel l'élément allongé ne prend pas de forme prédéterminée, si bien que la deuxième configuration est aléatoire, **caractérisé en ce que** ledit élément allongé (70) comprend des reliefs pour assurer une étanchéité renforcée entre l'élément allongé et la lumière, sélectionnés entre une configuration en vis sans fin (42), une configuration en spirale (53) et une pluralité de billes (64) et **en ce que** l'élément allongé (70) est imbibé d'au moins une composition thérapeutique, un agent pouvant gonfler, un agent bioactif, un médicament ou un composé.

2. Système d'occlusion selon la revendication 1, comprenant en outre un dispositif de coupe (150, 160, 170, 180, 195) adapté pour couper l'élément allongé, le dispositif de coupe pouvant être ramené à un état prêt à couper sans qu'il faille retirer le dispositif d'occlusion d'un patient, dans lequel l'élément allongé (162) est administré à travers une lumière d'un cathéter (164), la lumière comportant une première ouverture (163) dans une paroi latérale du cathéter, et dans lequel le dispositif de coupe (160) comprend une partie tubulaire (165) concentrique avec une partie distale du cathéter (164) et comporte une deuxième ouverture dans une paroi latérale de la partie tubulaire.

3. Système d'occlusion selon la revendication 1, dans lequel l'élément allongé est imbibé d'un premier réactif et le système comprend un deuxième réactif configuré pour administrer hydrauliquement l'élément allongé.

4. Système d'occlusion selon la revendication 1, dans lequel l'élément allongé (80) est tubulaire et comprend une extrémité scellée (87), dans lequel l'élément allongé est imbibé d'un composé polyvalent.

5. Système d'occlusion selon la revendication 4, dans lequel l'élément allongé est configuré pour être administré sur un cathéter (85) et cette administration est animée hydrauliquement par l'infusion ou le débit de solution saline à travers le cathéter (85) dans la direction de l'extrémité scellée (87) de l'élément allongé (80).

6. Système d'occlusion selon la revendication 4, dans lequel l'élément allongé est configuré pour être administré sur un cathéter (85) et cette administration est animée hydrauliquement par l'infusion ou le débit d'un copolymère séquencé à travers le cathéter (85) dans la direction de l'extrémité scellée (87) de l'élément allongé (80).

7. Système d'occlusion selon la revendication 1, comprenant en outre des éléments allongés multiples (110) logés dans des lumières multiples (118) à l'intérieur d'au moins l'extrémité distale du cathéter (115).

8. Système d'occlusion selon la revendication 7, dans lequel les multiples éléments allongés ont une variété de longueurs et peuvent être déployés individuellement.
